(19) Europäisches Patentamt
European Patent Office
Office européen des brevets

(11) EP 3 950 067 A1

(12) **EUROPEAN PATENT APPLICATION**
published in accordance with Art. 153(4) EPC

(43) Date of publication:
**09.02.2022 Bulletin 2022/06**

(21) Application number: **20782306.3**

(22) Date of filing: **16.03.2020**

(51) International Patent Classification (IPC):
***A61P 43/00*** *(2006.01)* ***A61P 17/00*** *(2006.01)*
***A61K 36/185*** *(2006.01)* ***A61K 36/424*** *(2006.01)*
***A61K 36/535*** *(2006.01)* ***A61K 36/537*** *(2006.01)*
***A61K 36/738*** *(2006.01)* ***A61K 36/882*** *(2006.01)*
***A61K 31/352*** *(2006.01)* ***A61K 31/7048*** *(2006.01)*

(52) Cooperative Patent Classification (CPC):
**A61K 31/352; A61K 31/7048; A61K 36/185; A61K 36/424; A61K 36/535; A61K 36/537; A61K 36/738; A61K 36/882; A61P 17/00; A61P 43/00**

(86) International application number:
**PCT/JP2020/011415**

(87) International publication number:
**WO 2020/203217 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019067473**

(71) Applicant: **Suntory Holdings Limited**
**Osaka 530-8203 (JP)**

(72) Inventors:
- **KITAGAWA, Sayuri**
  **Soraku-gun, Kyoto 619-0284 (JP)**
- **WACHI, Hiroshi**
  **Tokyo 142-8501 (JP)**

(74) Representative: **Vossius & Partner**
**Patentanwälte Rechtsanwälte mbB**
**Siebertstraße 3**
**81675 München (DE)**

(54) **LTBP-1 EXPRESSION PROMOTING COMPOSITION AND METHOD FOR SCREENING FOR SUBSTANCE HAVING LTBP-1 EXPRESSION PROMOTING FUNCTION**

(57) The present invention aims to provide, for example, a composition for promoting LTBP-1 expression, which promotes LTBP-1 expression, and a method of screening for a substance having an LTBP-1 expression-promoting action. The present invention relates to, for example, a composition for promoting LTBP-1 expression, the composition containing: an extract of at least one plant selected from the group consisting of a rose, jiaogulan, herb-Robert, calamus, shiso, common sage, and star fruit, wherein the rose is a rose containing at least one of a rosacyanin compound or a rosadelphin compound.

FIG.1

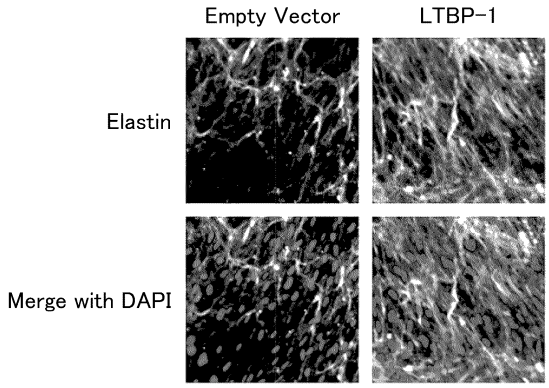


Magnification x200

Cell: NHDF−Neo

EP 3 950 067 A1

SEQUENCE LISTING FREE TEXT

SEQUENCE LISTING

<110>
\203T\203\223\203g\203\212\201[\203z\201[\203\213\203f\203B\203\223\203O\203X
\212\224\216®\211ï\216Ð Suntory Holdings Limited

<120>
\202k\202s\202a\202o\201|\202P\224-\214»\221£\220i\227p\221g\220¬\225¨\213y
\202Ñ\202k\202s\202a\202o\201|\202P\224-\214»\221£\220i\215ì\227p\202ð\227L
\202•\202é\225¨\216¿\202Ì\203X\203N\203\212\201[\203j\203\223\203O\225û\226@

<130> SP2019-0270

<150> JP2019-067473
<151> 2019-03-29

<160> 2

<170> PatentIn version 3.5

<210> 1
<211> 38
<212> DNA
<213> Artificial Sequence

<220>
<223> Primer (LTBP-1-Forward)

<400> 1
tgagcagtgt gtgaattctc ctggatctta ccagtgcg 38

<210> 2
<211> 34
<212> DNA
<213> Artificial Sequence

<220>
<223> Primer (LTBP-1-Reverse)

<400> 2
gactgcgctc gagctccagg tcactgtctt tctc 34

## Description

TECHNICAL FIELD

The present invention relates to, for example, a composition for promoting latent TGF-β binding protein (LTBP)-1 expression, a method of screening for a substance having an LTBP-1 expression-promoting action, and a method of promoting LTBP-1 expression.

BACKGROUND ART

Tissues such as skin, blood vessels, lungs, and ligaments require the property of returning to an initial form after being stretched, i.e., elasticity, for their functions. The elasticity in these tissues is maintained by tissues composed of cells and an extracellular matrix. Example of the extracellular matrix include collagen fibers, glycosaminoglycans (e.g., hyaluronic acid, keratan sulfate, heparan sulfate, chondroitin, chondroitin sulfate, and dermatan sulfate), and elastic fibers. Collagen fibers and glycosaminoglycans have great strength, but cannot be stretched or shrunk by external forces. In contrast, elastic fibers have elasticity. Thus, elastic fibers are the key responsible for elasticity in the tissues, such as skin, which require stretchability.

Elastic fibers are composed of crosslinked elastin deposited around fibers called microfibrils. Elastic fibers have very slow turnover. Breakdown or degeneration of elastic fibers by aging, ultraviolet light exposure, or the like causes arteriosclerosis, emphysema, skin sagging, or the like.

Recently, attention has been paid to intermolecular interactions between fibrin protein that promotes elastic fiber formation and latent TGF-β binding protein (LTBP). LTBP is a protein of the same superfamily as fibrillin. The LTBP family has four known members, i.e., LTBP-1, LTBP-2, LTBP-3, and LTBP-4, and characteristically has TGF-β binding 8-Cys domain. LTBP-2 is present without being bonded to latent TGF-β. In contrast, LTBP-1 and LTBP-3 are bonded to inactive latent TGF-β, and thus play the role of storing TGF-β in an inactive form so that TGF-β can be mobilized when needed. It has been reported that LTBP-4 is involved in elastic fiber formation through interactions with fibulin-5 (Patent Literature 1 and Non-Patent Literature 1).

CITATION LIST

- Patent Literature

Patent Literature 1: JP 6050573 B

- Non-Patent literature

Non-Patent Literature 1: Noda K et al., Proc Natl Acad Sci USA., 110, 2852-7, 2013

SUMMARY OF INVENTION

- Technical Problem

While involvement of the LTBP family in elastic fiber formation has been suggested, how LTBP-1 acts on elastic fiber formation is not much known. Thus, the present inventors focused on LTBP-1 and examined its effect on elastic fiber formation. As a result, as shown in Examples described later, it was found that, unlike LTBP-4, LTBP-1 has an action of promoting elastic fiber formation by itself. Thus, a substance that promotes LTBP-1 expression is considered to be useful for promoting elastic fiber formation. Yet, no reports have been made on a substance that promotes LTBP-1 expression.

The present invention aims to provide a composition for promoting LTBP-1 expression, which promotes LTBP-1 expression. The present invention also aims to provide, for example, a method of screening for a substance having an LTBP-1 expression-promoting action.

- Solution to Problem

As a result of extensive studies to achieve the above aims, the present inventors found that extracts of specific plants such as jiaogulan, herb-Robert, and roses containing at least one of a rosacyanin compound or a rosadelphin compound have an LTBP-1 expression-promoting action, and thus completed the present invention.

Specifically, the present invention encompasses, for example, the following compositions for promoting LTBP-1 expression.

(1) A composition for promoting LTBP-1 expression, the composition containing: an extract of at least one plant selected from the group consisting of a rose, jiaogulan, herb-Robert, calamus, shiso, common sage, and star fruit, wherein the rose is a rose containing at least one of a rosacyanin compound or a rosadelphin compound.
(2) The composition for promoting LTBP-1 expression according to (1) above, wherein the extract of the plant is an extract of at least one plant selected from the group consisting of jiaogulan, herb-Robert, and the rose containing at least one of a rosacyanin compound or a rosadelphin compound.
(3) The composition for promoting LTBP-1 expression according to (1) or (2) above, wherein the composition is intended for promoting LTBP-1 expression in skin.
(4) The composition for promoting LTBP-1 expression according to any one of (1) to (3) above, wherein the composition is used for promoting LTBP-1 expression decreased by ultraviolet light exposure in skin.
(5) The composition for promoting LTBP-1 expression according to any one of (1) to (4) above, wherein the composition is used for preventing or alleviating elastic fiber reduction due to ultraviolet light exposure by promoting LTBP-1 expression in skin.
(6) The composition for promoting LTBP-1 expression according to any one of (1) to (5) above, wherein the composition is a topical skin preparation.
(7) The composition for promoting LTBP-1 expression according to any one of (1) to (6) above, wherein the composition is a cosmetic product.
(8) The composition for promoting LTBP-1 expression according to any one of (1) to (5) above, wherein the composition is a food or beverage.
(9) A method of screening for a substance having an LTBP-1 expression-promoting action, the method including:

irradiating skin cells with ultraviolet light *in vitro;*
culturing the skin cells irradiated with ultraviolet light separately in a test medium containing a test substance and in a control medium not containing the test substance; and
comparing a level of LTBP-1 expression in test skin cells cultured in the test medium to a level of LTBP-1 expression in control skin cells cultured in the control medium,
wherein the test substance is selected as a substance having an LTBP-1 expression-promoting action when the level of LTBP-1 expression in the test skin cells is higher than the level of LTBP-1 expression in the control skin cells.

(10) A method of promoting LTBP-1 expression, the method including: applying an extract of at least one plants selected from the group consisting of a rose, jiaogulan, herb-Robert, calamus, shiso, common sage, and star fruit to a subject, wherein the rose is a rose containing at least one of a rosacyanin compound or a rosadelphin compound.
(11) Use of an extract of at least one plant selected from the group consisting of a rose, jiaogulan, herb-Robert, calamus, shiso, common sage, and star fruit for promoting LTBP-1 expression, wherein the rose is a rose containing at least one of a rosacyanin compound or a rosadelphin compound.

- Advantageous Effects of Invention

The present invention can provide a composition for promoting LTBP-1 expression, which promotes LTBP-1 expression. The present invention can promote LTBP-1 expression. The present invention can provide, for example, a method of screening for a substance having an LTBP-1 expression-promoting action.

BRIEF DESCRIPTION OF DRAWINGS

Fig. 1 is a set of photomicrographs showing results of fluorescent immunostaining evaluation of elastic fiber formation in LTBP-1-overexpressing dermal fibroblasts (magnification: ×200).
Fig. 2 is a graph showing results of semi-quantification of the amount of elastic fibers in LTBP-1-overexpressing dermal fibroblasts.

DESCRIPTION OF EMBODIMENTS

<Composition for promoting LTBP-1 expression>

The composition for promoting LTBP-1 expression of the present invention contains an extract of at least one plant selected from the group consisting of jiaogulan, herb-Robert, calamus, shiso, common sage, star fruit, and a rose containing at least one of a rosacyanin compound or a rosadelphin compound.

The composition for promoting LTBP-1 expression of the present invention contains the plant extract as an active ingredient. The active ingredient used in the composition for promoting LTBP-1 expression of the present invention may include an extract of any one of the above plants or extracts of two or more of the above plants.

In the present invention, jiaogulan refers to *Gynostemma pentaphyllum* (scientific name) of the genus *Gynostemma* of the family Cucurbitaceae. Herb-Robert refers to *Geranium robertianum* L. (scientific name) of the genus *Geranium* of the family Geraniaceae. Calamus refers to *Acorus calamus* (scientific name) of the genus *Acorus* of the family Acoraceae. Shiso refers to *Perilla frutescens* var. *crispa* (scientific name) of the genus *Perilla* of the family Lamiaceae. Common sage refers to *Salvia officinalis* (scientific name) of the genus *Salvia* of the family Lamiaceae. Star fruit refers to *Averrhoa carambola* (scientific name) of the genus *Averrhoa* of the family Oxalidaceae. Rose refers to a plant of the genus *Rosa* of the family Rosaceae, preferably a rose of the genus *Rosa* of the family Rosaceae, such as *Rosa hybrida* (scientific name).

The rose in the present invention is a rose containing at least one of a rosacyanin compound or a rosadelphin compound. The rose is not limited as long as it is a rose containing at least one of a rosacyanin compound or a rosadelphin compound. Preferably, it is a rose containing a rosacyanin compound and a rosadelphin compound.

The rosacyanin compound is at least one compound selected from the group consisting of rosacyanin A1, rosacyanin A2, and rosacyanin B. A rose containing a rosacyanin compound is not limited along as it is a rose containing at least one of the above compounds. Preferably, it is a rose containing the rosacyanin A1, the rosacyanin A2, and the rosacyanin B. The rosacyanin A1 is a compound represented by the following formula (I) in which $R^1$ is a hydrogen atom. The rosacyanin A2 is a compound represented by the following formula (II) in which $R^2$ is a hydrogen atom. The rosacyanin B is a compound represented by the following formula (III) in which $R^3$ is a hydrogen atom.

[Chem. 1]

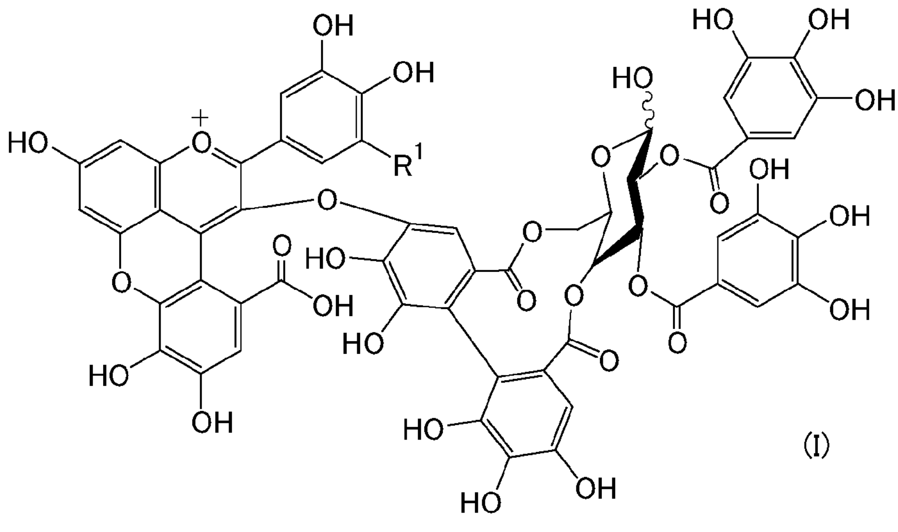

(I)

In the above formula, the coordination (wavy line) to the hydroxy group at position 1 of glucose indicates tautomerism of α-form and β-form; and $R^1$ represents a hydrogen atom or a hydroxy group.

[Chem. 2]

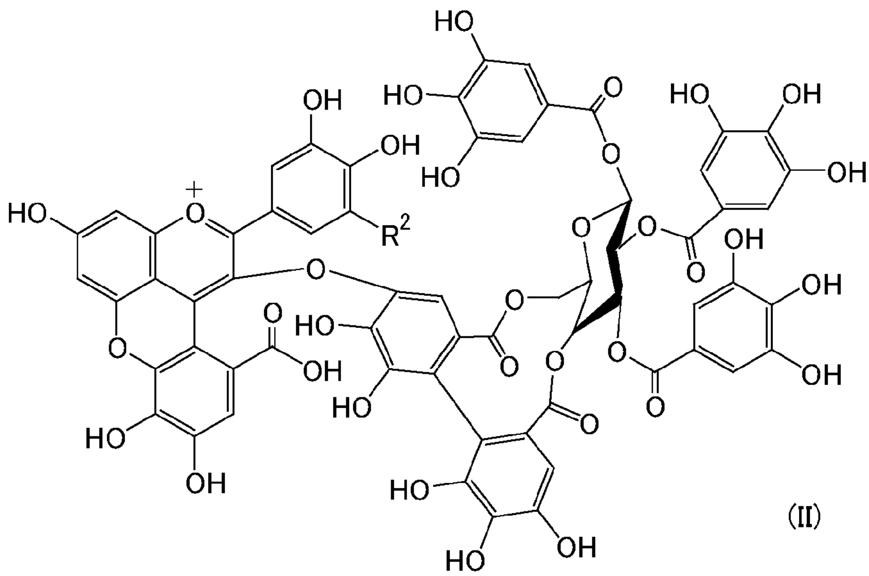

(II)

In the above formula, $R^2$ represents a hydrogen atom or a hydroxy group.

[Chem. 3]

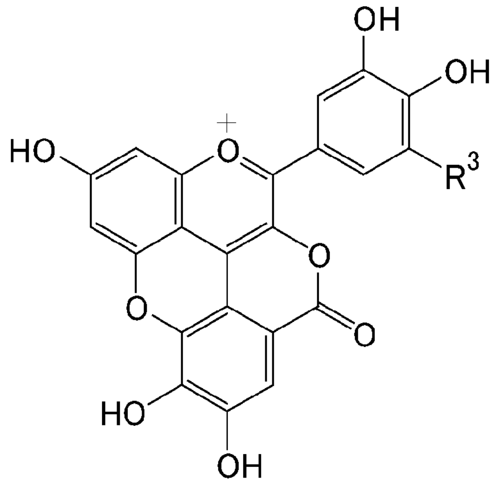

(III)

In the above formula, $R^3$ represents a hydrogen atom or a hydroxy group.

Examples of the rose containing a rosacyanin compound include blue-hued roses and mauve-hued roses. Examples of such roses include varieties such as Madame Violet, Purple Rain, Lavande, Manhattan Blue, Chantilly Lace, Blue Moon, Tasogare, Charles de Gaulle, Violet Dolly, Blue Ribbon, Aozora, Lady X, Blue Bajou, and Sterling Silver.

The rosadelphin compound is at least one compound selected from the group consisting of rosadelphin A1, rosadelphin A2, and rosadelphin B. The rose containing a rosadelphin compound is not limited as long as it is a rose containing at last one of the above compounds. Preferably, it is a rose containing the rosadelphin A1, the rosadelphin A2, and the rosadelphin B. The rosadelphin A1 is a compound represented by the above formula (I) in which $R^1$ is a hydroxy group. The rosadelphin A2 is a compound represented by the above formula (II) in which $R^2$ is a hydroxy group. The rosadelphin B is a compound represented by the above formula (III) in which $R^3$ is a hydroxy group.

The rosadelphin compounds are not found in wild roses. They are compounds produced by binding of gallate or tellimagrandin to delphinidin (a blue pigment) synthesized in a rose having a gene encoding flavonoid 3',5'-hydroxylase (hereinafter also referred to as "flavonoid 3',5'-hydroxylase gene") introduced thereinto by a method such as genetic engineering or a rose having flavonoid 3',5'-hydroxylase gene obtainable using a genetically modified rose as a parent.

Examples of the rose containing the rosadelphin compound(s) include roses having flavonoid 3',5'-hydroxylase gene introduced thereinto by a method such as genetic engineering and roses having flavonoid 3',5'-hydroxylase gene

obtainable using such a genetically modified rose as a parent. Examples of such roses include rose varieties such as APPLAUSE®. Petals of APPLAUSE® are known to contain the rosacyanin A1, the rosacyanin A2, and the rosacyanin B, as well as the rosadelphin A1, the rosadelphin A2, and the rosadelphin B (e.g., WO 2010/110382). APPLAUSE® refers to the same variety as SUNTORY blue rose APPLAUSE®, which is available from Suntory Flowers Co., Ltd. (Tokyo, Japan). A rose having flavonoid 3',5'-hydroxylase gene can also be produced by the method described in WO 2010/110382, for example. The flavonoid 3',5'-hydroxylase gene may be a plant-derived gene described in WO 2004/020637. For example, flavonoid 3',5'-hydroxylase gene derived from a pansy of the genus *Viola (Viola spp.* cv. black pansy) or the like is preferred. All the academic literature and patent literature described in this description are incorporated herein by reference.

The rose containing at least one of a rosacyanin compound or a rosadelphin compound in the present invention is preferably a rose containing at least one of a rosacyanin compound or a rosadelphin compound, more preferably a rose containing a rosacyanin compound and a rosadelphin compound in its petals. The presence of a rosacyanin compound and/or rosadelphin compound in a rose can be confirmed by HPLC-TOF-MS, for example, by the method described in WO 2010/110382, WO 2015/178385, or the like.

The plant extract in the present invention can be prepared using any part of the plants described above, such a root, rhizome, stem, leaf, branch, bark, flower, seed (including seed coat, germ, albumen, hypocotyl, cotyledon, and the like), fruit (including pulp and pericarp), or a combination of these parts. The plant extract can be produced by extraction with a solvent from any of these parts of the plants.

The following parts can be mentioned as examples of preferred parts for producing the plant extract. Preferred examples of the plant extract in the present invention include extracts of the following parts of the plants.

For jiaogulan, shiso, common sage, and star fruit, use of leaves is preferred. For herb-Robert, use of whole plants is preferred. For the rose containing at least one of a rosacyanin compound or a rosadelphin compound, use of petals is preferred. For calamus, use of rhizomes and/or roots is preferred, and use of rhizomes is more preferred.

These parts of the plants may be directly used for extraction, or may be crushed, cut, or dried and then used for extraction. The plant extract in the present invention may be an extract extracted from any of the above plants directly, or may be an extract extracted from a crushed, cut, or dried product of any of the above plants. An extract extracted from a crushed, cut, or dried product of any of the above plants is preferred.

In one embodiment of the present invention, the plant extract is preferably an extract of at least one plant selected from the group consisting of jiaogulan, herb-Robert, and the rose containing at least one of a rosacyanin compound or a rosadelphin compound, more preferably one selected from the group consisting of a leaf extract of jiaogulan, a whole plant extract of herb-Robert, and a petal extract of the rose containing at least one of a rosacyanin compound or a rosadelphin compound.

The plant extraction method is not limited. The plant extract can be obtained from the plant(s) by an extraction method usually used for extraction of plant components. The extraction method can be suitably adjusted, and extraction conditions are also not limited. For example, preferably, the plant extract is extracted with a solvent from any of the parts of the plants at room temperature or under heat. Operations such as stirring may be suitably performed during extraction.

A solvent (extraction solvent) to be used for plant extraction may be suitably selected, and one usually used to extract plant components can be used. Examples of the extraction solvent include water; C1-C5 lower monohydric alcohols such as methanol, ethanol, propanol, butanol, and pentanol; polyols such as ethylene glycol, propylene glycol, 1,2-butylene glycol, 1,3-butylene glycol, 1,4-butylene glycol, and 2,3-butylene glycol; ketones such as acetone and methyl ethyl ketone; esters such as methyl acetate and ethyl acetate; open-chain or cyclic ethers such as tetrahydrofuran and diethyl ether; polyethers such as polyethylene glycol; and squalane. Any of these may be used alone, or a solvent mixture of two or more thereof may be used. The lower monohydric alcohol is preferably a C1-C4 monohydric alcohol, more preferably a C2-C4 monohydric alcohol, still more preferably ethanol or the like. The polyol is preferably, for example, a diol or a triol, more preferably a diol. The polyol is preferably, for example, a C2-C4 polyol, more preferably 1,3-butylene glycol or the like. In particular, the extraction solvent is preferably water, a lower monohydric alcohol, a polyol, squalane, or a solvent mixture of two or more thereof; more preferably water, a lower monohydric alcohol, a polyol, or a solvent mixture of two or more thereof. In one embodiment, the extraction solvent is more preferably, for example, water, a lower monohydric alcohol or its aqueous solution, a polyol or its aqueous solution, squalane, or the like; still more preferably water, ethanol, an aqueous ethanol solution, 1,3-butylene glycol, an aqueous 1,3-butylene glycol solution, squalane, or the like. Preferably, the plant extract in the present invention is an extract extracted with any of the solvents described above. An acid or alkali may be added during extraction to adjust the pH of the extraction solvent.

Preferably, the extraction is followed by removal of plant residues (the plant or its parts after extraction) from the resulting liquid extract. The method of removing plant residues from the liquid extract is not limited. For example, a known separation means such as filtration or centrifugation can be used.

In one embodiment, preferably, the jiaogulan extract is a water extract of jiaogulan. Preferably, the herb-Robert extract, shiso extract, and star fruit extract are each a 1,3-butylene glycol extract or aqueous 1,3-butylene glycol extract

of the corresponding plant. Preferably, the calamus extract, common sage extract, and rose extract are each an ethanol extract or aqueous ethanol extract of the corresponding plant.

The following method can be used as an example of the plant extraction method. A crude plant or a dried plant is finely crushed, and an extraction solvent having a mass 5 to 20 times that of the crushed product is added for extraction at normal pressure and room temperature preferably for 10 minutes to 15 days, more preferably 30 minutes to 10 days, still more preferably 1 hour to 7 days, or at a temperature near the boiling point of the extraction solvent preferably for about 10 minutes to 1 day (more preferably for 10 minutes to 2 hours), followed by filtration to obtain a filtrate. The extraction may be performed under standing still or suitable stirring. The resulting liquid filtrate (liquid plant extract) may be directly used as an extract of the plant, or may be concentrated or dried as needed.

In the present invention, the liquid plant extract obtained by extraction can be directly used as a plant extract. The liquid plant extract may be diluted with a suitable solvent and used as a plant extract or may be concentrated or dried to obtain a concentrate or a dried powder. Alternatively, the liquid plant extract may be used in the form of a paste. The concentration method and the drying method are also not limited. Known methods such as freeze drying or spray drying can be used. Alternatively, the dried powder may be used in the form of a solution or suspension in a solvent.

In addition, the liquid plant extract or its concentrate or dried powder can be further purified by deodorizing, decolorizing, or the like, as needed, as long as the effect of the present invention is not impaired. Such a purification method may be any means usually used. Examples include filtration and various chromatographic techniques such as column chromatography using ion exchange resin, synthetic adsorption resin, activated carbon, or the like as a carrier. The plant extract in the present invention encompasses liquid extracts extracted with various solvents which are obtained by the extraction method described above; diluted solutions, concentrates, and dried powders of these liquid extracts; and purified products thereof. The plant extract can also be a commercial product.

The plant extract used in the present invention has an LTBP-1 expression-promoting action. In the present invention, the promotion of LTBP-1 expression includes promotion of LTBP-1 mRNA expression and promotion of LTBP-1 protein expression. The promotion of expression may be *in vivo* promotion of expression or *in vitro* promotion of expression in a culture system or the like. LTBP-1 is expressed preferably in skin cells, more preferably in human skin cells. Preferably, the composition for promoting LTBP-1 expression of the present invention is used as a composition for promoting LTBP-1 expression in the skin. The term "LTBP-1 expression" can also be referred to as "LTBP-1 production".

As shown in Examples described later, while ultraviolet light exposure decreases LTBP-1 expression in skin cells, use of the plant extract resulted in increased LTBP-1 expression as compared to non-use of the plant extract. Thus, the plant extract has an action of promoting LTBP-1 expression decreased by ultraviolet light exposure in the skin. In one embodiment, the composition for promoting LTBP-1 expression of the present invention can be used for promoting LTBP-1 expression decreased by ultraviolet light exposure in the skin. Promoting LTBP-1 expression that has been decreased can be understood as restoring LTBP-1 expression. Promoting LTBP-1 expression can also suppress the decrease in LTBP-1 expression caused by ultraviolet light exposure or the like.

As shown in Examples, LTBP-1 has an action of promoting elastic fiber formation by itself. Thus, the substance having the action of promoting LTBP-1 expression has the action of promoting elastic fiber formation.

In the present invention, the term "the action of promoting elastic fiber formation" refers to an action of promoting deposition and crosslinking of elastin around microfibrils. The degree of deposition and the degree of crosslinking are not limited. In the present invention, when use of a substance promoted deposition of elastin around microfibrils to some degree as compared to non-use of the substance, the substance is considered to have the action of promoting elastic fiber formation.

The plant extract used in the composition for promoting LTBP-1 expression of the present invention has an LTBP-1 expression-promoting action. Thus, for example, the composition for promoting LTBP-1 expression of the present invention can be used for promoting elastic fiber formation. For example, promoting LTBP-1 expression in the skin promotes elastic fiber formation in the skin. The composition for promoting LTBP-1 expression of the present invention is useful for preventing or ameliorating conditions or symptoms for which promotion of LTBP-1-involved elastic fiber formation is desired (e.g., elastic fiber denaturation or elastic fiber reduction caused by aging or ultraviolet light exposure). In one embodiment, the composition for promoting LTBP-1 expression of the present invention promotes LTBP-1 expression in the skin, whereby the composition can be used for preventing or alleviating elastic fiber reduction due to ultraviolet light exposure. The composition for promoting LTBP-1 expression of the present invention is also useful for cosmetic purposes such as prevention or amelioration of skin sagging and prevention or amelioration of wrinkles by the LTBP-1 expression-promoting action. The term "preventing" or "prevention" encompasses prevention of onset, delay of onset, lower incidence, and the like. The term "alleviating", "ameliorating", or "amelioration" encompasses alleviation of symptoms, suppression of progression of symptoms, cure of symptoms, and the like.

The composition for promoting LTBP-1 expression of the present invention can also be used as a reagent for culturing cells with an ability to regenerate elastic fibers. The composition of the present invention can also be used for promoting artificial elastic fiber formation. The composition can also be used for producing artificial tissues such as

artificial skin, skin models, and artificial blood vessels.

The form of the composition for promoting LTBP-1 expression of the present invention is not limited. Examples include powders, granules, pastes, solids, and liquids, and the form can be suitably selected. Other component(s) can be added, in addition to the plant extract, to the composition of the present invention, as long as the effect of the present invention is not impaired. Examples of the other component(s) include those that can be used in products described later, such as cosmetic products (cosmetics), food and beverages, pharmaceutical products, and quasi-pharmaceutical products.

For example, the composition for promoting LTBP-1 expression of the present invention can be provided in the form of an agent, but the form is not limited to the agent. Such an agent can be directly provided as a composition, or can be provided as a composition containing the agent. In one embodiment, the composition for promoting LTBP-1 expression of the present invention can also be referred to as an "LTBP-1 expression promoter".

The composition for promoting LTBP-1 expression of the present invention is applicable for both therapeutic use (medical use) and non-therapeutic use (non-medical use). The "non-therapeutic" is a concept that does not include medical activities, i.e., a concept that does not include methods of surgery, therapy, or diagnosis of humans.

The composition for promoting LTBP-1 expression of the present invention can be used in various applications such as cosmetic products, food and beverages, pharmaceutical products, and quasi-pharmaceutical products. The composition for promoting LTBP-1 expression of the present invention may be a cosmetic product, food or beverage, pharmaceutical product, or quasi-pharmaceutical product for promoting LTBP-1 expression by itself, or may be a formulation, material, or the like used by being added to the cosmetic product, food or beverage, pharmaceutical product, quasi-pharmaceutical product, or the like.

For example, the composition for promoting LTBP-1 expression of the present invention is suitably used as a topical skin preparation. The topical skin preparation includes cosmetic products, pharmaceutical products, and quasi-pharmaceutical products. Preferably, the topical skin preparation is a cosmetic product. In one embodiment, the composition for promoting LTBP-1 expression when used for promoting LTBP-1 expression in the skin is preferably a topical skin preparation, more preferably a cosmetic product.

The composition for promoting LTBP-1 expression of the present invention can also be used as a pharmaceutical product or quasi-pharmaceutical product other than the topical skin preparation.

In another embodiment, preferably, the composition for promoting LTBP-1 expression is a food or beverage. The following describes the case where the composition for promoting LTBP-1 expression of the present invention is a topical skin preparation such as a cosmetic product, a pharmaceutical product, or a quasi-pharmaceutical product, a food or beverage, or the like.

When the composition for promoting LTBP-1 expression of the present invention is a topical skin preparation (a topical skin preparation for promoting LTBP-1 expression), the dosage form or the like is not limited. For example, any form such as a solution, emulsion, cream, gel, powder, aerosol, mist, capsule, or sheet may be used. Preferably, the topical skin preparation is a cosmetic product. The product form of the cosmetic product is also not limited. Examples include skin care cosmetic products such as face wash, makeup remover, skin lotion, essence lotion, face pack, emulsion, cream, and sunscreen lotion products; makeup cosmetic products such as foundation, makeup base, lipstick, eyeshadow, eyeliner, mascara, eyebrow pencil, brush, and nail enamel products; hair cosmetic products such as shampoo, hair conditioner, hair styling products, hair dyes, and hair growth products; cleansing agents such as soap and body wash; and bath salts.

The plant extract may be used alone in the cosmetic product. In addition, such a cosmetic product may suitably contain one or more cosmetically acceptable carriers, additives, and like other components, as long as the effect of the present invention is not impaired. Examples include water, alcohols, oils, surfactants, thickeners, metal soaps, gelling agents, powders, chelating agents, water-soluble polymers, film forming agents, resins, inclusion compounds, antimicrobial agents, deodorants, salts, pH adjusting agents, ultraviolet light absorbers, extracts of microorganisms/plants/animals other than those mentioned above, keratolytic agents, enzymes, hormones, other vitamins, humectants, antiseptics, anti-inflammatory agents, and perfumes. The cosmetic product can be produced by a common production method in which, for example, the plant extract is mixed with one or more cosmetically acceptable components described above, and the mixture is then processed into a desired form.

When the topical skin preparation is a pharmaceutical product or quasi-pharmaceutical product, the plant extract may be used alone, or the plant extract may be used in combination with pharmaceutically or quasi-pharmaceutically acceptable carriers, additives, and like other components. Examples of such components include excipients, binders, disintegrants, lubricants, antioxidants, and colorants.

One or more of these components can be used as needed.

The composition for promoting LTBP-1 expression of the present invention can be a pharmaceutical product or quasi-pharmaceutical product other than the topical skin preparation. Such a pharmaceutical product or quasi-pharmaceutical product may be administered orally or parenterally. The pharmaceutical or quasi-pharmaceutical product can be provided in the form of a formulation for oral administration (agent for internal use) or a formulation for parenteral

administration. Examples of the dosage form of the formulation for oral administration include liquids, tablets, powders, fine granules, granules, sugar-coated tablets, capsules, suspensions, emulsions, and chewable tablets. Examples of the dosage form of the formulation for parenteral administration include injections and infusions. In such a pharmaceutical product or quasi-pharmaceutical product, the plant extract may be used alone, or the plant extract may be used in combination with pharmaceutically or quasi-pharmaceutically acceptable carriers, additives, and like other components. Examples of such components include excipients, binders, disintegrants, lubricants, antioxidants, colorants, and flavoring substances. One or more of these components can be used as needed. The pharmaceutical product or quasi-pharmaceutical product can be produced by a common production method in which, for example, the plant extract is mixed with one or more pharmaceutically or quasi-pharmaceutically acceptable components, and the mixture is then processed into a desired form.

When the composition for promoting LTBP-1 expression of the present invention is a food or beverage, the food or beverage is not limited. Examples include general foods, general beverages, health foods, foods with function claims, and foods for specified health uses. The form of the food or beverage is also not limited. The health foods, foods with function claims, and foods for specified health uses can be provided in various forms of formulations such as liquids, tablets, powders, fine granules, granules, sugar-coated tablets, capsules, suspensions, emulsions, chewable tablets, and liquid foods.

In the food or beverage, the plant extract may be used alone, or the plant extract may be used in combination with ingredients acceptable in food and beverages, such as other food or beverage materials and additives for food and beverages, to provide a food or beverage for promoting LTBP-1 expression (a food or beverage composition for promoting LTBP-1 expression). Such a food and beverage can be produced by a common production method. For example, the plant extract is added to a food or beverage material or the like in the production of the food or beverage.

When the composition for promoting LTBP-1 expression of the present invention is in any of the forms such as a topical skin preparation, a pharmaceutical product or quasi-pharmaceutical product other than the topical skin preparation, or a food or beverage, the amount of the plant extract in the composition, for example, as the mass of the extract in terms of dry matter, is preferably 0.0000001 to 100 mass%, more preferably 0.000001 to 100 mass%, still more preferably 0.000001 to 99.9 mass%, yet still more preferably 0.00001 to 99.9 mass%, particularly preferably 0.00001 to 10 mass%. When the composition contains two or more plant extracts, the above amount is the total amount of these plant extracts.

The amount of the composition for promoting LTBP-1 expression of the present invention to be used is not limited, and can be suitably set according to conditions, body weight, sex, age, or other factors of a subject. When the composition is a topical skin preparation such as a cosmetic product, the amount of the composition as the mass of the plant extract (in terms of dry matter) is, for example, preferably 0.01 to 500 mg per adult (60 kg) per day. When the composition is a pharmaceutical product or quasi-pharmaceutical product for oral administration, the dose of the composition as the mass of the plant extract (in terms of dry matter) is, for example, preferably 0.01 to 500 mg per adult (60 kg) per day. When the composition is a food or beverage, the intake of the composition as the mass of the plant extract (in terms of dry matter) is, for example, preferably 0.01 to 500 mg per adult (60 kg) per day. The plant extract in the above amount can be ingested or administered at once or in several portions. The timing at which a topical skin preparation containing the plant extract is applied to the skin and the timing at which a food or beverage, pharmaceutical product, or quasi-pharmaceutical product containing the plant extract is ingested or administered are not limited. The composition for promoting LTBP-1 expression of the present invention can be used by various methods known per se, depending on its form, dosage form, and the like.

The applicable subject of the composition for promoting LTBP-1 expression of the present invention is not limited. The composition is applicable to humans, non-human mammals, or the like. Humans are preferred. For example, preferably, the applicable subject is one with decreased LTBP-1 expression or one wanting or needing to promote LTBP-1 expression.

<Method of screening for substance having LTBP-1 expression-promoting action>

The present invention also encompasses a method of screening for a substance having an LTBP-1 expression-promoting action, the method including: irradiating skin cells with ultraviolet light *in vitro;* culturing the skin cells irradiated with ultraviolet light separately in a test medium containing a test substance and in a control medium not containing the test substance; and comparing a level of LTBP-1 expression in test skin cells cultured in the test medium to a level of LTBP-1 expression in control skin cells cultured in the control medium, wherein the test substance is selected as a substance having an LTBP-1 expression-promoting action when the level of LTBP-1 expression in the test skin cells is higher than the level of LTBP-1 expression in the control skin cells.

The screening method of the present invention is a method that is performed *in vitro* (an *in vitro* screening method).

Skin cells that can be used in the screening method of the present invention are skin cells of a human or non-human mammal in which LTBP-1 is expressed. Examples of the skin cells to be used in the method of the present invention include cultured skin cells such as normal human dermal fibroblasts. Culture conditions for growing the cells are not limited, and any known conditions may be employed depending on the cells.

In the screening method of the present invention, the skin cells are irradiated with ultraviolet light. The ultraviolet light irradiation can be, for example, ultraviolet light B (UVB) irradiation. In one embodiment, the cumulative dose can be, for example, 1 to 20 $mJ/cm^2$, preferably 3 to 10 $mJ/cm^2$.

The screening method of the present invention includes culturing skin cells irradiated with ultraviolet light in a medium containing a test substance (test medium) or in a medium not containing the test substance (control medium) .

Any test substance may be used. Examples include liquid plant extracts, cell extracts, fermentation products, proteins, peptides, vitamins, and synthetic compounds. These may be known substances or novel substances. The test medium can be prepared by adding a test substance to a medium. The control medium may be the same as the test medium, except that no test substance is added.

The medium, culture conditions, and the like can be suitably set according to the cell type and the like. The culture time is not limited, and may be suitably set according to culture conditions and the like. For example, after ultraviolet light irradiation, the skin cells may be cultured preferably for 5 to 72 hours, more preferably 12 to 48 hours, in a medium containing a test substance or a control medium.

An example of such a medium may be $\alpha$-MEM medium. The medium may contain commercially available serum. The medium may be a commercial product.

In the present invention, after ultraviolet light irradiation, the skin cells are cultured in a test medium containing a test substance or a control medium not containing the test substance. After culturing, the level of LTBP-1 expression in test skin cells cultured in the test medium is compared to the level of LTBP-1 expression in control skin cells cultured in the control medium. The test substance is selected as a substance having an LTBP-1 expression-promoting action when the level of LTBP-1 expression in the test skin cells is higher than the level of LTBP-1 expression in the control skin cells.

The ultraviolet light irradiation decreases the level of LTBP-1 expression in the skin cells. The test skin cells irradiated with ultraviolet light are cultured in the test medium containing a test substance, and the control skin cells irradiated with ultraviolet light are cultured in the control medium not containing the test substance. Then, the level of LTBP-1 expression is compared between the test skin cells and the control skin cells. This makes it possible to screen for a substance that can exert the LTBP-1 expression-promoting action, for example, in the skin with decreased LTBP-1 expression caused by ultraviolet light exposure or the like.

The level of LTBP-1 expression in the skin cells can be determined by measuring the amount of mRNA encoding LTBP-1 or the amount of LTBP-1 protein. The amount of mRNA or protein can be measured by a known method.

For example, the amount of LTBP-1 protein can be measured by immunochemical assay, mass spectrometry, or the like. Examples of the immunochemical assay include enzyme linked immunosorbent assay (ELISA) and western blotting. The amount of protein can also be measured using a commercially available ELISA kit (available from MyBio-Source) or the like.

The amount of mRNA encoding LTBP-1 can be measured by PCR, northern blotting, microarray, live imaging with a fluorescent probe, or the like.

The base sequence and amino acid sequence (ORF) of human LTBP-1 are known. The base sequence (mRNA) is deposited in the public database GenBank with Accession No. Q 14766. A person skilled in the art can easily design or synthesize primers for measuring the amount of mRNA encoding LTBP-1 and amplify DNA, for example.

The screening method of the present invention may include culturing skin cells before ultraviolet light irradiation. In culturing before ultraviolet light irradiation, a test medium containing the test substance may be used. In one embodiment, before ultraviolet light irradiation, the skin cells may be cultured in a test medium containing the test substance or in a control medium not containing the test substance. Preferably, in this case, in culturing after ultraviolet light irradiation, the skin cells cultured in a test medium containing the test substance before ultraviolet light irradiation are cultured in the test medium, and the skin cells cultured in a control medium before ultraviolet light irradiation are cultured in the control medium. When the skin cells cultured in the test medium before ultraviolet light irradiation have a higher level of LTBP-1 expression than the skin cells cultured in the control medium, i.e., when the test substance has the LTBP-1 expression-promoting action, the test substance suppresses the decrease in LTBP-1 expression caused by ultraviolet light irradiation. In one embodiment, in order to select a test substance that suppresses the decrease in LTBP-1 expression in skin cells caused by ultraviolet light exposure, preferably, the skin cells are cultured in a test medium containing the test substance before ultraviolet light irradiation. The media, culture conditions, and the like for culturing before ultraviolet light irradiation can be suitably set according to the cell type and the like. The above-described culture conditions and the like can be used.

In another embodiment, the screening method of the present invention includes culturing skin cells separately in a test medium containing a test substance and in a control medium not containing the test substance; irradiating test skin cells cultured in the test medium and control skin cells cultured in the control medium with ultraviolet light; and

comparing a level of LTBP-1 expression in test skin cells cultured in the test medium to a level of LTBP-1 expression in control skin cells cultured in the control medium, wherein the test substance is selected as a substance having an LTBP-1 expression-promoting action when the level of LTBP-1 expression in the test skin cells is higher than the level of LTBP-1 expression in the control skin cells. The media, culture conditions, ultraviolet light irradiation conditions, and the like are not limited, and those described above can be used.

<Method of promoting LTBP-1 expression, and the like>

The present invention also encompasses a method of promoting LTBP-1 expression, including applying an extract of at least one plant selected from the group consisting of jiaogulan, herb-Robert, calamus, shiso, common sage, star fruit, and a rose containing at least one of a rosacyanin compound or a rosadelphin compound to a subject.

In an example of a preferred embodiment, the present invention provides a method of promoting LTBP-1 expression in the skin. The subject is the same as the applicable subject of the composition for promoting LTBP-1 expression described above. Humans or non-human mammal are preferred, and humans are more preferred.

The plant extract may be applied to (used on) the subject by a method such as oral administration or parenteral administration of the plant extract. Parenteral administration is preferred, and a method of applying to the skin is more preferred. Preferably, the plant extract is applied to the skin when used for promoting LTBP-1 expression in the skin. The method may be therapeutic or non-therapeutic. In one embodiment, the method of the present invention may be a method of promoting LTBP-1 expression decreased by ultraviolet light exposure in the skin, the method including applying the plant extract to a subject after the subject is exposed to ultraviolet light.

The present invention also encompasses use of an extract of at least one plant selected from the group consisting of jiaogulan, herb-Robert, calamus, shiso, common sage, star fruit, and a rose containing at least one of a rosacyanin compound or a rosadelphin compound for promoting LTBP-1 expression.

Preferably, the use is for promoting LTBP-1 expression in the skin. Preferably, the use is for humans or non-human mammals. In one embodiment, preferably, the use is use of the plant extract for promoting LTBP-1 expression decreased by ultraviolet light exposure in the skin. The use may be therapeutic or non-therapeutic.

The plant extract in the method and use, its preferred embodiment, its preferred amount to be used, and the like are as described above for the composition for promoting LTBP-1 expression. One plant extract may be used, or two or more plant extracts may be used. The plant extract(s) may be used directly, or may be used in combination with other components. The plant extract can be used in the form of the above-described topical skin preparation, food or beverage, or the like.

The present invention also encompasses use of the plant extract for producing a composition for promoting LTBP-1 expression. Preferred embodiments and the like of the composition for promoting LTBP-1 expression and the plant extract are as described above.

EXAMPLES

The following provides Examples that more specifically describe the present invention. The present invention is not limited to these Examples.

<Test Example 1>

(Production of LTBP-1-overexpressing dermal fibroblasts)

mRNA was extracted from cultured normal human dermal fibroblasts (NHDF-Neo: purchased from Lonza) using RNAzol® RT (Cosmo Bio Co., Ltd.). With the extracted mRNA as a template, reverse transcription reaction was performed using M-MLV reverse transcriptase. With the resulting cDNA as a template, PCR reaction was performed using PrimeSTAR® GXL DNA Polymerase (Takara Bio Inc.).

The following primers were used to amplify a DNA fragment (LTBP-1 cDNA) encoding LTBP-1.

(Primer set for LTBP-1)

LTBP-1-Forward: 5'-TGAGCAGTGTGTGAATTCTCCTGGATCTTACCAGTGCG-3' (SEQ ID NO: 1)

LTBP-1-Reverse: 5'-GACTGCGCTCGAGCTCCAGGTCACTGTCTTTCTC-3' (SEQ ID NO: 2)

(Production of plasmid)

The amplified gene was electrophoresed on ethidium bromide-containing 2% agarose gel. A single band was observed under UV irradiation, and the band portion was cut out. The DNA fragment encoding LTBP-1 was purified using Wizard® SV Gel and PCR Clean-UP System (Promega KK.), and was then inserted into pMXs-Puro Retroviral Vector using DNA Ligation kit Mighty Mix (Takara Bio Inc.). The LTBP-1 cDNA was added to the purified pMXs-Puro Retroviral Vector (100 ng) at a molar ratio of 1:3, and DNA Ligation kit Mighty Mix (Takara Bio Inc.) was added for overnight reaction. After completion of the reaction, the resulting mixture was added to E. coli DH5 $\alpha$ competent cells (Takara Bio Inc.) for incubation, followed by addition of SOC medium and stirring. The resulting reaction solution was applied to ampicillin-containing LB agar medium, and cultured overnight at 37°C. After culturing, colonies formed on a plate were scraped off with an end of a tip, and added to the LB medium. The colonies were shakecultured overnight at 37°C. After culturing, Fast Gene® Plasmid Mini Kit (Nippon Genetics Co., Ltd.) was used for purification. Thus, a plasmid was obtained. Further, the plasmid and the DNA fragment encoding LTBP-1 amplified with the primer set of SEQ ID NO: 1 and SEQ ID NO: 2 were cut with EcoRI and XhoI, followed by ligation, transformation, and purification, whereby an intended plasmid was obtained. The purified plasmid was cut with EcoRI and XhoI. Subsequently, electrophoresis was performed on 2% agarose gel to confirm insertion of the gene.

(Introduction of plasmid)

Plat-A cells were cultured overnight in a plasmid-containing medium obtained by adding pMXs-Puro Retroviral Vector, the produced plasmid for LTBP-1, FuGENE HD Transfection Reagent (Promega KK.), and 10% serum to Opti-MEM I medium. After culturing, the cells were cultured in a 10%-serum containing medium, whereby a retrovirus-containing medium was produced. Neonatal normal human dermal fibroblasts were treated in the produced retrovirus-containing medium to produce LTBP-1-overexpressing dermal fibroblasts. To provide control, pMXs-Puro Retroviral Vector without insertion of LTBP-1 cDNA (an empty vector) was introduced into the neonatal normal human dermal fibroblasts. Thus, control cells were produced.

Hereinafter, the resulting LTBP-1-overexpressing dermal fibroblasts are also referred to as "L1L-NHDF", and the dermal fibroblasts with insertion of the empty vector are also referred to as "control cells".

<Test Example 2>

Using the LTBP-1-overexpressing dermal fibroblasts (L1L-NHDF) and control cells produced in Test Example 1, the effect of LTBP-1 to promote elastic fiber formation was evaluated.

(1) Fluorescent immunostaining

The L1L-NHDF and control cells were separately seeded onto 8-well chamber slides at $4 \times 10^4$ cells/well and cultured in a 10%-serum containing medium for five days. After fixation with paraformaldehyde, the cells were fluorescence immunostained with a specific primary antibody and a specific secondary antibody, and were observed under a confocal laser scanning microscope. The primary antibody was an antibody that recognizes human elastin, and the secondary antibody was an IgG antibody labeled with fluorescence.

(2) Semi-quantification of the amount of fibers by ELISA

The L1L-NHDF and control cells were separately seeded onto a 96-well plate at $1.8 \times 10^4$ cells/well and cultured in a 10%-serum containing medium for five days. After fixation with paraformaldehyde, to the cells were added a primary antibody and a secondary antibody and then a TMB (3,3',5,5'-tetramethylbenzidine) substrate reagent to measure the absorbance (measurement wavelength: 450 nm; control wavelength: 600 nm) of each well with a microplate reader. The primary antibody was an antibody that recognizes human elastin, and the secondary antibody was an IgG antibody labeled with HRP.

(Results)

(1) Elastic fiber formation in LTBP-1-overexpressing dermal fibroblasts (L1L-NHDF)

Elastic fiber formation in the L1L-NHDF was determined by fluorescent immunostaining by the above method.

Fig. 1 is a set of photomicrographs showing results of fluorescent immunostaining evaluation of elastic fiber formation in the L1L-NHDF. The upper row shows elastin (elastic fibers) immunostained with fluorescence. The lower row shows cell nuclei immunostained with fluorescence. In Fig. 1, "Empty Vector" (left column) represents the control cells, and "LTBP-1" (right column) represents the LTBP-1-overexpressing dermal fibroblasts. The results of fluorescent immunostaining show a tendency of increase in elastic fiber formation in the L1L-NHDF as compared to the control cells (Fig. 1).

(2) Semi-quantification of the amount of elastic fibers in L1L-NHDF

Elastic fiber formation in the L1L-NHDF was semi-quantified by ELISA. Fig. 2 shows the results. The vertical axis in Fig. 2 is the absorbance at 450 nm. An unpaired two-tailed student's t-test was performed to test the significant difference (**$P < 0.01$ ($n = 4$)). In Fig. 2, "Empty Vector" represents the control cells, and "LTBP-1" represents the LTBP-1-overexpressing dermal fibroblasts. Fig. 2 shows a significant increase in elastic fiber formation in the L1L-NHDF as compared to the control cells.

The above results show that overexpression of LTBP-1 promoted elastic fiber formation. This shows that LTBP-1 has an action of promoting elastic fiber formation by itself.

<Test Example 3>

Whether ultraviolet light exposure changes the level of LTBP-1 expression in the cells was examined.

Neonatal normal human dermal fibroblasts (NHDF-NB: purchased from Kurabo Industries Ltd.) were seeded onto a 100-mm culture dish and cultured to confluence in 5 vol% carbon dioxide concentration at 37°C in $\alpha$-MEM medium (available from Sigma-Aldrich). Subsequently, the culture supernatant was removed. The medium was replaced with a control medium (a-MEM medium containing 0.1 mass% dimethyl sulfoxide (DMSO)), and the cells were cultured for 24 hours. Then, the cells were irradiated with ultraviolet light B (UVB) (cumulative dose: about 5.7 $mJ/cm^2$), and further cultured for 24 hours. After culturing, the cells (the cells irradiated with UVB: UVB (+)) were collected and used as specimens. mRNA was extracted from the cells (specimens) using RNeasy Mini Kit (Qiagen), and the level of LTBP-1 gene expression was quantified by RT-PCR.

For comparison, the method described above was used to culture neonatal normal human dermal fibroblasts (NHDF-NB), except that no UVB irradiation was performed. The same method was used to extract mRNA from the cells (the cells not irradiated with UV: UVB (-)), and cDNA was synthesized. Then, the level of LTBP-1 gene expression was quantified by RT-PCR.

RT-PCR was performed by TaqMan Assay available from Applied Biosystems, using commercially available primers for LTBP-1 (available from Applied Biosystems). The PCR reaction was performed at 95°C for 20 seconds and at 60°C 20 seconds. This cycle was repeated 40 times.

The test was repeated three times (Test Nos. 1 to 3). In each test, six specimens ($n = 6$) were prepared. The level of LTBP-1 gene expression was measured twice per specimen. The results were averaged to obtain a measured value. Such measured values ($n = 6$) were averaged to obtain a quantification result of the level of LTBP-1 expression.

Table 1 shows the results. For each test, the level of LTBP-1 expression (%) is expressed as the level of LTBP-1 expression (the amount of mRNA) in the cells irradiated with UV (UVB (+)) relative to the level of LTBP-1 expression (the amount of mRNA) in the cells not irradiated with UV (UVB (-)) taken as 100%.

[Table 1]

| UV (+) | Level of LTBP-1 expression (%) |
|---|---|
| Test No. 1 | 76.34 |
| Test No. 2 | 74.08 |
| Test No. 3 | 71.16 |
| Average | 73.86 |

Table 1 shows that the level of LTBP-1 gene expression in the cells irradiated with UVB was lower by an average of 26.1% than the level of expression in the cells not irradiated with UVB. This shows that irradiation of human dermal fibroblasts with UVB decreases LTBP-1 expression.

<Preparation Example 1>

Preparation of plant-derived extract

Various plants were screened to select the following seven plants, and extracts were obtained from these plants by extraction with a solvent. Plants and parts used for extraction are as follows.

Jiaogulan (*Gynostemma pentaphyllum*): leaves
Herb-Robert (*Geranium robertianum*): whole plants
Rose (*Rosa hybrida,* variety APPLAUSE®, Suntory Flowers Co., Ltd.): petals
Shiso (*Perilla frutescens* var. *Crispa*): leaves
Calamus (*Acorus calamus*): rhizomes
Common sage (*Salvia officinalis*): leaves
Star fruit (*Averrhoa carambola*): leaves

The rose (variety "APPLAUSE") is a blue-hued rose containing the rosacyanin A1, the rosacyanin A2, and the rosacyanin B, as well as the rosadelphin A1, the rosadelphin A2, and the rosadelphin B in its petals.

Crushed products of the above plants were immersed in a solvent for extraction. More specifically, in the case of jiaogulan, leaves were used for extraction with water, and the resulting liquid extract was filtered to obtain a filtrate. The filtrate was concentrated and formed into a powder, and then the powder was dissolved in 1,3-butylene glycol and water, followed by filtration. Thus, a liquid jiaogulan extract was obtained. In the case of herb-Robert, whole plants were used for extraction with 1,3-butylene glycol to obtain a liquid extract. In the case of rose, petals were used for extraction with ethanol to obtain a liquid extract. In the case of shiso, leaves were used for extraction with 1,3-butylene glycol to obtain a liquid extract. In the case of calamus, rhizomes were used for extraction with ethanol to obtain a liquid extract. In the case of common sage, leaves were used for extraction with ethanol to obtain a liquid extract. In the case of star fruit, leaves were used for extraction with 1,3-butylene glycol to obtain a liquid extract. Plant residues were removed by filtration from each of the resulting liquid extracts. Each filtrate was obtained as a liquid plant extract.

Each of the resulting liquid plant extracts was concentrated in an evaporator, and then freeze dried. Thus, the extracts of the plants were obtained in the form of a powder. These powdered plant extracts (jiaogulan extract, herb-Robert extract, rose extract, shiso extract, calamus extract, common sage extract, and star fruit extract) were used for the following evaluation.

In Examples 1 to 3 below, six specimens (n = 6) were prepared for each sample. The level of LTBP-1 gene expression was measured twice per specimen. The results were averaged to obtain a measured value. Measured values of these specimens (n = 6) were averaged to obtain a quantification result of the level of LTBP-1 expression. The same applies to the control. A student's t test was performed to test the significant difference.

<Example 1>

The LTBP-1 expression-promoting action of each plant-derived extract in neonatal normal human dermal fibroblasts was evaluated, using the level of LTBP-1 expression as an index.

Neonatal normal human dermal fibroblasts (NHDF-NB: purchased from Kurabo Industries Ltd.) were seeded onto a 100-mm culture dish and cultured to confluence in 5 vol% carbon dioxide concentration at 37°C in α-MEM medium (available from Sigma-Aldrich). Subsequently, the culture supernatant was removed. The medium was replaced with a sample-containing medium, and the cells were cultured for 24 hours. Then, the cells were irradiated with ultraviolet light B (UVB) (cumulative dose: about 5.7 mJ/$cm^2$), and were further cultured for 24 hours. After culturing, the cells were collected and used as specimens. mRNA was extracted from the cells by the same method as in Test Example 3, and the level of LTBP-1 gene expression was quantified by RT-PCR.

Each sample-containing medium was prepared by adding one of the plant extracts to the α-MEM medium. Table 2 shows the plant extracts used and the concentrations of the plant extracts in the respective media. Each plant extract (powder) was dissolved in DMSO and added such that the final concentration of DMSO in the medium was 0.1 mass%.

Separately, UVB irradiation and culturing were performed by the methods described above, except that the sample-containing medium was replaced by the control medium (a-MEM medium containing 0.1 mass% DMSO) used in Test Example 3. The resulting cells were used as control. The control was also subjected to quantification of the level of LTBP-1 gene expression by the same method described above.

Table 2 shows the results. The level of LTBP-1 expression is expressed as the level of LTBP-1 expression (the amount of mRNA) obtained with the plant extract relative to the level of LTBP-1 expression (the amount of mRNA) in the control taken as 100%. The significant difference is the result of the test relative to the control.

[Table 2]

| Sample | Level of LTBP-1 expression (%) | Significant difference |
|---|---|---|
| Jiaogulan extract 10 μg/m L | 132.53 | < 0.05 |
| Herb-Robert extract 20 μg/m L | 118.52 | < 0.05 |
| Rose extract 10 μg/mL | 114.38 | < 0.05 |
| Rose extract 20 μg/mL | 120.03 | < 0.01 |

The level of LTBP-1 expression was significantly higher when the cells were cultured in the media separately containing the jiaogulan extract, the herb-Robert extract, and the rose extract than when the cells were cultured in the media not containing these extracts (control). This shows that these plant extracts have the LTBP-1 expression-promoting action.

<Example 2>

Neonatal normal human dermal fibroblasts (NHDF-NB: purchased from Kurabo Industries Ltd.) were seeded onto a 100-mm culture dish and cultured to confluence in 5 vol% carbon dioxide concentration at 37°C in α-MEM medium (available from Sigma-Aldrich). Subsequently, the culture supernatant was removed. The medium was replaced with fresh α-MEM medium, and the cells were cultured for 24 hours. Then, the medium was removed and replaced with Hanks' balanced salt solution (HBSS). Subsequently, the cells were irradiated with UVB (cumulative dose: about 5.7 $mJ/cm^2$). After UV irradiation, the HBSS was replaced with a sample-containing medium, and the cells were further cultured for 24 hours. After culturing, the cells were collected and used as specimens. mRNA was extracted from the cells by the same method as in Test Example 3, and the level of LTBP-1 gene expression was quantified by RT-PCR.

The sample-containing medium was prepared by adding one of the plant extracts to the α-MEM medium. Table 3 shows the plant extracts used and the concentration of each plant extract in the medium. Each plant extract was dissolved in DMSO and added such that the final concentration of DMSO in the medium was 0.1 mass%. Separately, UVB irradiation and culturing were performed by the methods described above, except that the sample-containing medium was replaced by the control medium (a-MEM medium containing 0.1 mass% DMSO) used in Test Example 3. Then, the resulting cells were used as control (+UV). Culturing was performed by the same method described above, except that the sample-containing medium was replaced by the control medium used in Test Example 3 and that no UVB irradiation was performed. The resulting cells were used as control (-UV). These controls were also subjected to quantification of the level of LTBP-1 gene expression by the method described above.

Table 3 shows the results. In Table 3, "Control (-UV)" is the control not irradiated with UVB, and "Control (+UV)" is the control irradiated with UVB. In Table 3, "-" in UV irradiation means that no UVB irradiation was performed, and "+" means that UV irradiation was performed. The significant difference is the significant difference of the level of LTBP-1 expression (%) of each sample relative to the "Control (+UV)".

The level of LTBP-1 expression (%) of each sample is expressed as the value (%) relative to the level of LTBP-1 expression (the amount of mRNA) in the control irradiated with UVB (Control (+UV)) taken as 100%. The degree of restoring effect of the plant extract on the decrease in the level of LTBP-1 expression caused by UVB irradiation is shown as the "restoration rate (%)". The restoration rate (%) was determined by the following calculation from the level of LTBP-1 expression (C0) in the control (-UV) not irradiated with UV, the level of LTBP-1 expression (C1) in the control irradiated with UV, and the level of LTBP-1 expression (S) in the cells to which the plant extract was added.

$$\text{Restoration rate (\%)} = 100 \times ((S)-(C1))/((C0)-(C1))$$

[Table 3]

| Sample | UV irradiation | Level of LTBP-1 expression (%) | Restoration rate (%) | Significant difference |
|---|---|---|---|---|
| Control (-UV) | - | 242.96 | - | - |
| Control (+UV) | + | 100.00 | 0.00 | - |
| Jiaogulan extract 10 μg/mL | + | 118.39 | 12.86 | <0.01 |

(continued)

| Sample | UV irradiation | Level of LTBP-1 expression (%) | Restoration rate (%) | Significant difference |
|---|---|---|---|---|
| Jiaogulan extract 20 μg/mL | + | 121.01 | 14.70 | <0.01 |
| Herb-Robert extract 5 μg/mL | + | 120.72 | 14.49 | <0.05 |
| Herb-Robert extract 20 μg/mL | + | 112.80 | 8.96 | <0.05 |

The jiaogulan extract and the herb-Robert extract restored the LTBP-1 expression that was decreased by UV exposure.

<Example 3>

The LTBP-1 expression-promoting action was evaluated by the same method as in Example 1, except that the plant extracts shown in Table 4 were used. Table 4 shows the concentrations of the plant extracts in the sample-containing media and evaluation results. The level of LTBP-1 expression is expressed as the level of LTBP-1 expression (the amount of mRNA) obtained with the plant extract relative to the level of LTBP-1 expression (the amount of mRNA) in the control taken as 100%. The significant difference is the result of the test relative to the control. Table 4 shows that the shiso extract, calamus extract, common sage extract, and star fruit extract have the LTBP-1 expression-promoting action.

[Table 4]

| Sample | Level of LTBP-1 expression (%) | Significant difference |
|---|---|---|
| Shiso extract 10 μg/mL | 117.17 | < 0.05 |
| Calam us extract 5 μg/mL | 123.96 | < 0.05 |
| Calam us extract 10 μg/mL | 123.06 | < 0.05 |
| Calam us extract 20 μg/mL | 138.59 | < 0.01 |
| Common sage extract 5 μg/mL | 114.12 | < 0.05 |
| Common sage extract 10 μg/mL | 113.88 | < 0.01 |
| Common sage extract 20 μg/mL | 115.41 | < 0.01 |
| Star fruit extract 10 μg/m L | 120.83 | < 0.01 |
| Star fruit extract 20 μg/m L | 115.96 | < 0.01 |

## Claims

**1.** A composition for promoting LTBP-1 expression, the composition comprising:

an extract of at least one plant selected from the group consisting of a rose, jiaogulan, herb-Robert, calamus, shiso, common sage, and star fruit, wherein the rose is a rose containing at least one of a rosacyanin compound or a rosadelphin compound.

**2.** The composition for promoting LTBP-1 expression according to claim 1, wherein the extract of the plant is an extract of at least one plant selected from the group consisting of jiaogulan, herb-Robert, and the rose containing at least one of a rosacyanin compound or a rosadelphin compound.

**3.** The composition for promoting LTBP-1 expression according to claim 1 or 2, wherein the composition is intended for promoting LTBP-1 expression in skin.

**4.** The composition for promoting LTBP-1 expression according to any one of claims 1 to 3,

wherein the composition is used for promoting LTBP-1 expression decreased by ultraviolet light exposure in skin.

**5.** The composition for promoting LTBP-1 expression according to any one of claims 1 to 4,
wherein the composition is used for preventing or alleviating elastic fiber reduction due to ultraviolet light exposure by promoting LTBP-1 expression in skin.

**6.** The composition for promoting LTBP-1 expression according to any one of claims 1 to 5,
wherein the composition is a topical skin preparation.

**7.** The composition for promoting LTBP-1 expression according to any one of claims 1 to 6,
wherein the composition is a cosmetic product.

**8.** The composition for promoting LTBP-1 expression according to any one of claims 1 to 5,
wherein the composition is a food or beverage.

**9.** A method of screening for a substance having an LTBP-1 expression-promoting action, the method comprising:

irradiating skin cells with ultraviolet light *in vitro;*
culturing the skin cells irradiated with ultraviolet light separately in a test medium containing a test substance and in a control medium not containing the test substance; and
comparing a level of LTBP-1 expression in test skin cells cultured in the test medium to a level of LTBP-1 expression in control skin cells cultured in the control medium,
wherein the test substance is selected as a substance having an LTBP-1 expression-promoting action when the level of LTBP-1 expression in the test skin cells is higher than the level of LTBP-1 expression in the control skin cells.

**10.** A method of promoting LTBP-1 expression, the method comprising:
applying an extract of at least one plant selected from the group consisting of a rose, jiaogulan, herb-Robert, calamus, shiso, common sage, and star fruit to a subject, wherein the rose is a rose containing at least one of a rosacyanin compound or a rosadelphin compound.

**11.** Use of an extract of at least one plant selected from the group consisting of a rose, jiaogulan, herb-Robert, calamus, shiso, common sage, and star fruit for promoting LTBP-1 expression,
wherein the rose is a rose containing at least one of a rosacyanin compound or a rosadelphin compound.

FIG.1

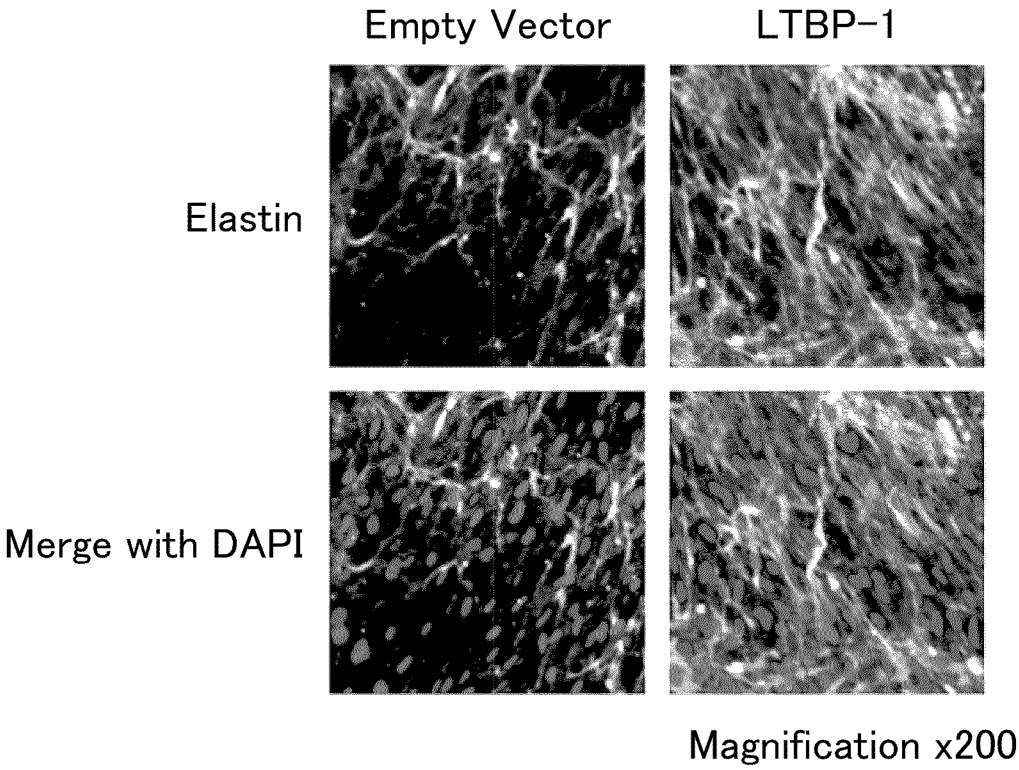


Cell: NHDF-Neo

FIG.2

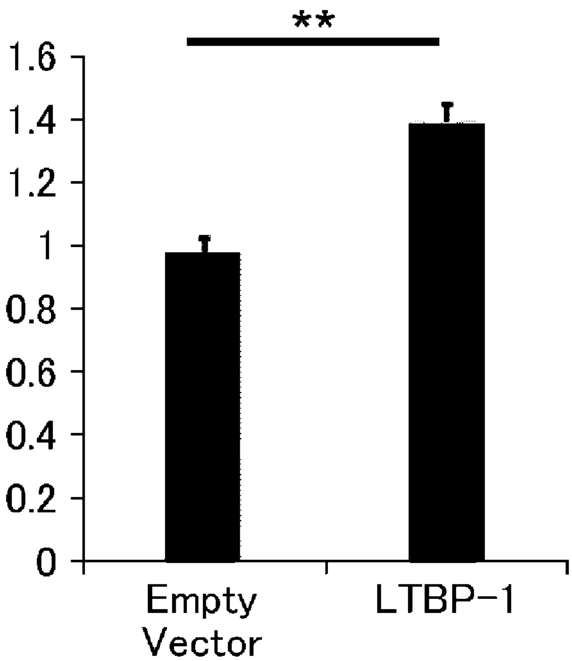

**INTERNATIONAL SEARCH REPORT**

International application No.
PCT/JP2020/011415

A. CLASSIFICATION OF SUBJECT MATTER
Int. Cl. A61P43/00(2006.01)i, A61P17/00(2006.01)i, A61K36/185(2006.01)i, A61K36/424(2006.01)i, A61K36/535(2006.01)i, A61K36/537(2006.01)i, A61K36/738(2006.01)i, A61K36/882(2006.01)i, A61K31/352(2006.01)i, A61K31/7048(2006.01)i
FI: A61K36/424, A61K36/185, A61K36/882, A61K36/535, A61K36/537, A61K36/738, A61K31/7048, A61K31/352, A61P17/00, A61P43/00 105

According to International Patent Classification (IPC) or to both national classification and IPC

B. FIELDS SEARCHED
Minimum documentation searched (classification system followed by classification symbols)
Int. Cl. A61P43/00, A61P17/00, A61K36/185, A61K36/424, A61K36/535, A61K36/537, A61K36/738, A61K36/882, A61K31/352, A61K31/7048

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | WO 2015/178385 A1 (SUNTORY HOLDINGS LTD.) 16 November 2015, claims, examples, paragraphs [0002]-[0004] | 1-8, 10, 11 |
| X | JP 2010-241777 A (ICHIMARU PHARCOS INC.) 28 October 2010, claims, examples, (in particular, production example 1), paragraph [0010] | 1-8, 10, 11 |
| X | JP 2003-95858 A (NONOGAWA SHOJI YK) 03 April 2003, claims, examples, (in particular, examples 2, 8, table 2, paragraph [0040]), paragraphs [0002], [0003] | 1-8, 10, 11 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13.05.2020 | 26.05.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/011415

C (Continuation). DOCUMENTS CONSIDERED TO BE RELEVANT

| Category* | Citation of document, with indication, where appropriate, of the relevant passages | Relevant to claim No. |
|---|---|---|
| X | JP 2005-2057 A (ICHIMARU PHARCOS INC.) 06 January 2005, claims, examples, (production examples 1-4, fig. 3-5, tests 3-5), [0024], [0005]-[0007] | 1-8, 10, 11 |
| X | JP 2007-186457 A (ICHIMARU PHARCOS INC.) 26 July 2007, claims, paragraph [0028], table 2, paragraphs [0116], [0016]-[0019] | 1-8, 10, 11 |
| X | JP 2014-129267 A (KOSE CORP.) 10 July 2014, claims, example 1, fig. 2, example 13, paragraphs [0003], [0004], [0007] | 1-8, 10, 11 |
| X | WO 2018/124002 A1 (SUNTORY HOLDINGS LTD.) 05 July 2018, claims, examples, (in particular, preparation example 1, (2), (5), (9), paragraph [0097], tables 1-3), paragraphs [0002], [0003], [0010] | 1-8, 10, 11 |
| X | JP 2008-520588 A (BIOPHARMACOPAE DESIGN INTERNATIONAL INC.) 19 June 2008, claims, paragraph [0221], tables, "Acorus calamus", "Salvia officinalis" | 1-8, 10, 11 |
| X | JP 11-12122 A (POLA CHEMICAL INDUSTRIES INC.) 19 January 1999, claims, paragraph [0009], table 1, paragraph [0017], table 3, paragraph [0002], example 38 | 1-8, 10, 11 |
| X | JP 2002-226323 A (MARUZEN PHARMACEUTICALS CO., LTD.) 14 August 2002, claims, examples, (in particular, production example 2, test examples 1-4), paragraphs [0003]-[0005] | 1-8, 10, 11 |
| X | SHERRATT, Michael J, et al., Low-dose ultraviolet radiation selectively degrades chromophore-rich extracellular matrix components, Journal of Pathology, 2010, vol. 222, no. 1, pp. 32-40, ISSN 0022-3417, <DOI: 10.1002/ path.2730>, abstract, fig. 4 | 9 |

Form PCT/ISA/210 (continuation of second sheet) (January 2015)

**INTERNATIONAL SEARCH REPORT**
Information on patent family members

International application No.
PCT/JP2020/011415

| Patent Documents referred to in the Report | Publication Date | Patent Family | Publication Date |
|---|---|---|---|
| WO 2015/178385 A1 | 16.11.2015 | US 2017/0079904 A1 claims, examples, paragraphs [0002]-[0004]<br>EP 3146957 A1<br>KR 10-2017-0007812 A<br>CN 106456492 A | |
| JP 2010-241777 A | 28.10.2010 | (Family: none) | |
| JP 2003-95858 A | 03.04.2003 | (Family: none) | |
| JP 2005-2057 A | 06.01.2005 | (Family: none) | |
| JP 2007-186457 A | 26.07.2007 | (Family: none) | |
| JP 2014-129267 A | 10.07.2014 | (Family: none) | |
| WO 2018/124002 A1 | 05.07.2018 | CN 110022853 A<br>TW 201828918 A | |
| JP 2008-520588 A | 19.06.2008 | US 2007/0122492 A1<br>WO 2006/053415 A1 claims, page 75, paragraph [0003], tables, Acorus calamus, Salvia officinalis<br>US 2011/0311661 A1<br>EP 1819356 A1 | |
| JP 11-12122 A | 19.01.1999 | (Family: none) | |
| JP 2002-226323 A | 14.08.2002 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6050573 B
- WO 2010110382 A
- WO 2004020637 A
- WO 2015178385 A


### Non-patent literature cited in the description


- **NODA K et al.** *Proc Natl Acad Sci USA.,* 2013, vol. 110, 2852-7